# EUROPÄISCHE PATENTSCHRIFT

(11) **EP 3 497 089 B1**
(45) Veröffentlichungstag und Bekanntmachung des Hinweises auf die Patenterteilung: **29.07.2020**
(21) Anmeldenummer: 17749189.1
(22) Anmeldetag: 07.08.2017
(51) Int. Cl.: C07D 307/79, C07D 307/82, A61K 31/343

(54) **VERFAHREN ZUR HERSTELLUNG VON 4-SUBSTITUIERTEN 2,3-DIHYDRO-1-BENZOFURANDERIVATEN DURCH ZYKLISIERUNG VON 2-(2-DIAZONIUM-6-SUBSTITUIERTEN-PHENYL)ETHANOL SALZEN**
PROCESS FOR THE PREPARATION OF 4-SUBSTITUTED 2,3-DIHYDRO-1-BENZOFURANE DERIVATIVES BY CYCLISATION OF 2-(2-DIAZONIUM-6-SUBSTITUTED-PHENYL)ETHANOL SALTS
PROCÉDÉ POUR LA PRÉPARATION DE DÉRIVÉS DE 2,3-DIHYDRO-1-BENZOFURANE SUBSTITUÉS EN POSITION 4 PAR CYCLISATION DE SÉLS 2-(2-DIAZONIUM-6-SUBSTITUÉ-PHÉNYL)ÉTHANOL

(30) Priorität: 12.08.2016 EP 16183949
(43) Veröffentlichungstag der Anmeldung: 19.06.2019
(73) Patentinhaber: Bayer CropScience Aktiengesellschaft, 40789 Monheim am Rhein (DE)
(72) Erfinder: GALLENKAMP, Daniel, 42113 Wuppertal (DE); FORD, Mark, James, 65207 Wiesbaden-Breckenheim (DE)
(74) Vertreter: BIP Patents
(86) Internationale Anmeldenummer: PCT/EP2017/069933
(87) Internationale Veröffentlichungsnummer: WO 2018/029141

(56) Entgegenhaltungen:
- WO-A1-2004/052851
- WO-A1-2009/062285
- WO-A2-2014/091167
- ZHU J ET AL: "Copper(I)-catalyzed intramolecular cyclization reaction of 2-(2'-chlorophenyl)ethanol to give 2,3-dihydrobenzofuran", TETRAHEDRON LETTERS, PERGAMON, GB, Bd. 41, Nr. 21, 1. Mai 2000 (2000-05-01), Seiten 4011-4014, XP004204482, ISSN: 0040-4039, DOI: 10.1016/S0040-4039(00)00548-7
- BAKKE, JAN M.; ROHOLDT, HAEGE M.: "Synthesis of 2,3-Dihydrobenzofuran", ACTA CHEMICA SCANDINAVICA, SERIES B: ORGANIC CHEMISTRY AND BIOCHEMISTRY, Bd. 34b, Nr. 1, 1980, Seiten 73-73, XP002761357, DOI: 10.3891/acta.chem.scand.34b-0073
- G. M. BENNETT AND MOSTAFA MAHMOUD HAFEZ: "52. A synthesis of dihydroindole, dihydrothionaphthen, and dihydrobenzofuran", JOURNAL OF THE CHEMICAL SOCIETY, 1941, Seiten 287-288, XP002761358, DOI: 10.1039/JR9410000287

## Beschreibung

Die vorliegende Erfindung betrifft ein Verfahren zur Herstellung von substituierten 2,3-Dihydro-1-benzofuran-Derivaten.

Substituierte 2,3-Dihydrobenzofuran Derivate sind nützliche Zwischenstufen bei der Herstellung substituierter Styrol-Derivate, welche wiederum nützliche Zwischenstufen bei der Herstellung agrochemischer Wirkstoffe (siehe beispielsweise WO 2012/025557) sind. 4-Brom-2,3-dihydro-1-benzofuran ist aus WO 2014/091167 bekannt.

Ein mögliches Verfahren zur Herstellung von 4-Chlor-2,3-dihydro-1-benzofuran ist in der Literatur beschrieben. Die Herstellung erfolgt durch intramolekulare Zyklisierung von 2-(2,6-Dichlorphenyl)-ethanol unter Verwendung von 1.2 Equivalenten NaH in Gegenwart katalytischer Mengen CuCl *(*Tetrahedron Lett. 2000, 41, 4011) in Toluol bei 100 °C. Nachteilig bei diesem Verfahren ist, dass 2-(2,6-Dichlorphenyl)ethanol als Rohstoff nicht im technischen Maßstab verfügbar ist bzw. über eine mehrstufige kostenintensive Reaktionssequenz hergestellt werden muss, NaH als Base für den technischen Maßstab ungeeignet ist und Cu-Schwermetall Abfall anfällt.

Ein Verfahren zur Herstellung von 4-Methyl-2,3-dihydro-1-benzofuran ist aus WO 2009/062285 bekannt. Die Herstellung erfolgt über die Cyclisierung von 2-Bromo-1-(2-hydroxy-6-methyl-phenyl)-ethanon zu 4-Methylbenzofuran-3-on und anschließende Reduktion zum 4-Methyl-2,3-dihydro-1-benzofuran. Nachteilig ist, dass das Startmaterial für dieses Verfahren ebenfalls über eine aufwendige mehrstufige Reaktionssequenz hergestellt werden muss und die Ausbeute über zwei Stufen nur 42% beträgt.

Eine alternative und allgemeine Möglichkeit zur Darstellung von 2,3-Dihydrobenzofuranen besteht in der Zyklisierung der Diazonium Salz Verbindungen von 2-(2-Aminophenyl)ethanol Derivaten. Beispielsweise liefert die Behandlung von 2-(2-Aminophenyl)ethanol mit NaNO₂ und H₂SO4 in wässriger Lösung bei 0 °C und folgendem Erhitzen des Reaktionsgemisches auf 50 °C 2,3-Dihydrobenzofuran in einer Ausbeute von 50% (J. Chem. Soc. 1941, 287). Die Umsetzung ist unter identischen Bedingungen in Acta Chem. Scand. B 1980, B34, 73 beschrieben mit einer Ausbeute von 35% des 2,3-Dihydrobenzofurans sowie 50% 2-(2-Hydroxyethyl)phenol als Nebenkomponente. In WO 2004/052851 ist die Umsetzung von 2-(2-Amino-5-chlorphenyl)propan-1,3-diol zu (5-Chlor-2,3-dihydro-1-benzofuran-3-yl)methanol unter den oben beschriebenen Reaktionsbedingungen beschrieben ohne die Angabe einer Ausbeute. Nachteilig an diesem Verfahren ist, dass bei der Umsetzung von 2-(2-Amino-6-chlorphenyl)ethanol unter diesen Reaktionsbedingungen neben 4-Chlor-2,3-dihydro-1-benzofuran 3-Chlor-2-(2-hydroxyethyl)phenol als Nebenkomponente entsteht. Dadurch wird die Ausbeute an 4-Chlor-2,3-dihydro-1-benzofuran herabgesetzt und ein zusätzlicher Reinigungsschritt erforderlich. In J. Am. Chem. Soc. 2013, 135, 7086 ist die Umsetzung von 2'-Amino-3'-brombiphenyl-2-ol mit 1.2 Equivalenten NaNO₂ in einem Gemisch aus TFA/Wasser 20:1 bei 0 °C und anschließendem Erhitzen des Reaktionsgemisches auf 70 °C beschrieben, wodurch 4-Bromdibenzo[b,d]furan in einer Ausbeute von 75% isoliert werden kann. Ähnliche Reaktionsbedingungen sind in RSC Adv. 2015, 5, 44728 für die Zyklisierung (und simultane Nitrierung) von beispielsweise 2'-Amino-5-chlor-4'-methylbiphenyl-2-ol zu 2-Chlor-7-methyl-4-nitrodibenzo[b,d]furan beschrieben. Nachteilig an diesem Verfahren ist, dass bei der Umsetzung von 2-(2-Amino-6-chlorphenyl)ethanol unter diesen Reaktionsbedingungen neben 4-Chlor-2,3-dihydro-1-benzofuran (3-Chlor-2-(2-hydroxyethyl)phenyl-trifluoracetat als Nebenkomponente entsteht. Dadurch wird die Ausbeute an 4-Chlor-2,3-dihydro-1-benzofuran herabgesetzt und ein zusätzlicher Reinigungsschritt erforderlich.

Wegen der Bedeutung von substituierten 2,3-Dihydrobenzofuran-Derivaten als Baustein zur Synthese neuer agrochemischer Wirkstoffe besteht die Aufgabe darin, ein Verfahren zu finden, das großtechnisch und kostengünstig eingesetzt werden kann und die oben beschriebenen Nachteile umgeht. Auch ist es erstrebenswert, die speziellen 2,3-Dihydro-1-benzofuran-Derivate mit hoher Ausbeute und hoher Reinheit zu erhalten, so dass die Zielverbindung vorzugsweise keiner weiteren möglicherweise komplexen Aufreinigung unterzogen werden muss.

Diese Aufgabe wurde gelöst durch ein Verfahren zur Herstellung von substituierten 2,3-Dihydro-1-benzofuran-Derivaten der Formel **(I)**: 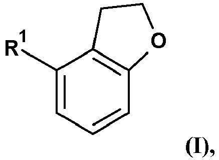 in der
- R¹: für Cl (**Ia**), Br (**Ib**) oder Methyl (**Ic**) steht,
dadurch gekennzeichnet dass ein Anilin der Formel (**II**) 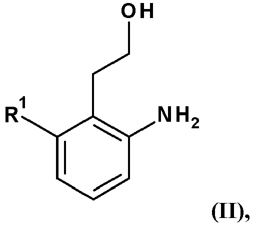 in der
R¹ für Cl, Br oder Methyl steht, in Gegenwart von organischem Nitrit und organischen oder anorganischen Säuren in organischen Lösungsmitteln zum Diazoniumsalz der Formel (**III**) umgesetzt wird, 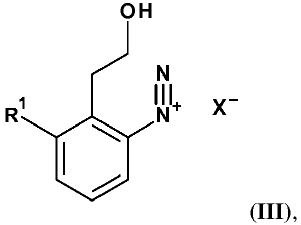 in der
- R¹: für Cl, Br oder Methyl steht,
- X⁻: für das Gegenion der organischen oder anorganischen Säure steht,
das durch Erhitzen zu Verbindungen der Formel (**I**) weiterreagiert.

Bevorzugt ist ein erfindungsgemäßes Verfahren, bei dem die Restedefinitionen der Formeln (**I**)**,** (**II**)**,**
und (**III**) wie folgt sind:
- R¹: steht für Cl,
- X⁻: steht für Cl⁻, HSO₄⁻, Cl₃COO⁻, F₃CCOO⁻.

### Beschreibung des Prozesses

Die erfindungsgemäße Reaktion ist in Schema 1 dargestellt.

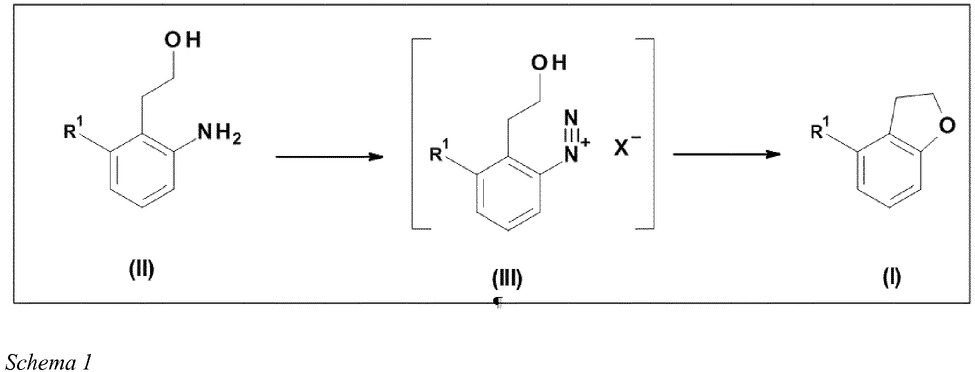

Die gewünschten 2,3-Dihydro-1-benzofuran-Derivate der allgemeinen Formel (**I**) werden mit dem erfindungsgemäßen Verfahren - Umsetzen des Anilins der Formel (**II**) zu Diazoniumsalz Verbindungen der Formel (**III**) in Anwesenheit von organischem Nitrit und anorganischen oder organischen Säuren in einem organischen Lösungsmittel und anschließende Weiterreaktion zu Verbindungen der Formel (**I**) durch Erhitzen - mit guten Ausbeuten und in hoher Reinheit erhalten.

Überraschenderweise werden durch die Verwendung von organischen Nitriten, vorzugsweise Alkylnitriten, in organischen Lösemitteln höhere Ausbeuten und Reinheiten erzielt und die Entstehung von Nebenkomponenten unterdrückt.

Als Lösungsmittel für das erfindungsgemäße Verfahren kommen grundsätzlich alle unter den Reaktionsbedingungen inerten organischen aprotischen Lösungsmittel oder Lösungsmittelgemische in Frage, unter anderem: Nitrile wie z.B. Acetonitril, Propionitril und Butyronitril; Kohlenwasserstoffe und halogenierte Kohlenwasserstoffe wie Hexan, Heptan, Cyclohexan, Methylcyclohexan, Toluol, ortho-Xylol, meta-Xylol, para-Xylol, Mesitylen, Chlorbenzol, ortho-Dichlorbenzol, Dichlormethan, 1,2-Dichlorethan, 1-Chlorbutan, Anisol oder Nitrobenzol. Bevorzugt wird das Lösungsmittel ausgewählt aus der Gruppe der Kohlenwasserstoffe und halogenierten Kohlenwasserstoffe: Hexan, Heptan, Cyclohexan, Methylcyclohexan, Toluol, ortho-Xylol, meta-Xylol, para-Xylol, Mesitylen, Chlorbenzol, ortho-Dichlorbenzol, Dichlormethan, 1,2-Dichlorethan, 1-Chlorbutan, Anisol, Nitrobenzol oder Gemische dieser Lösungsmittel oder der Nitrile: Acetonitril, Propionitril, Butyronitril. Besonders bevorzugt verwendet man 1,2-Dichlorethan, 1-Chlorbutan, Dichlormethan, Toluol, Xylol, Chlorbenzol, 1,2-Dichlorbenzol und/oder Acetonitril.

Als anorganische Säuren eignen sich Salzsäure und Schwefelsäure.

Als organische Säuren eignen sich Trifluoressigsäure (TFA) und Trichloressigsäure (TCA).

Als organische Nitrite eignen sich Alkylnitrite. Bevorzugt werden C₁-C₆-Alkylnitrite (wie z.B. Isopropylnitrit, n-Butylnitrit, Isobutylnitrit, tert-Butylnitrit, Pentylnitrit oder Isopentylnitrit) verwendet. Besonders bevorzugt werden organische Nitrite ausgewählt aus Isopropylnitrit, n-Butylnitrit, Isobutylnitrit, tert-Butylnitrit, Pentylnitrit und Isopentylnitrit verwendet. Ganz besonders bevorzugt werden n-Butylnitrit, tert-Butylnitrit und/oder Isopentylnitrit verwendet.

Besonders bevorzugt sind folgende Kombinationen aus den oben beschriebenen Gruppen der Lösungsmittel, organischen Nitriten und Säuren:
a) ein oder mehrere Nitrile als Lösungsmittel, ein oder mehrere C₁-C₆-Alkylnitrite als organisches Nitrit und Schwefelsäure oder Salzsäure als Säure;
b) Kohlenwasserstoffe und/oder halogenierte Kohlenwasserstoffe als Lösungsmittel, ein oder mehrere C₁-C₆-Alkylnitrite als organisches Nitrit und Trifluoressigsäure oder Trichloressigsäure als Säure.

Ganz besonders bevorzugt sind folgende Kombinationen aus den oben beschriebenen Gruppen der Lösungsmittel, organischen Nitriten und Säuren:
a) 1,2-Dichlorethan, 1-Chlorbutan, Dichlormethan, Chlorbenzol, Toluol, Xylol und/oder 1,2-Dichlorbenzol als Lösemittel in Kombination mit Trifluoressigsäure oder Trichloressigsäure als Säure in Kombination mit n-Butylnitrit, tert-Butylnitrit und/oder Isopentylnitrit als Nitrit;
b) Acetonitril, Propionitril und/oder Butyronitril als Lösemittel in Kombination mit Schwefelsäure oder Salzsäure als Säure in Kombination mit n-Butylnitrit, tert-Butylnitrit und/oder Isopentylnitrit als Nitrit.

Die Temperatur des erfindungsgemäßen Verfahrens kann in weiten Grenzen variiert werden. Üblicherweise arbeitet man zur Bildung der Diazoniumsalz Verbindungen der Formel (**III**) bei einer Temperatur von 0 °C bis 20 °C, vorzugsweise von 0 °C bis 10 °C. Besonders bevorzugt wird die Umsetzung bei einer Temperatur im Bereich von 0 °C bis 5 °C durchgeführt. Für die Weiterreaktion der Verbindungen der Formel (**III**) zu Verbindungen der Formel (**I**) arbeitet man üblicherweise bei einer Temperatur von 20 °C bis 80 °C, vorzugsweise bei 60 bis 80 °C

Üblicherweise wird das erfindungsgemäße Verfahren bei Normaldruck durchgeführt. Es ist auch möglich die Reaktion im Vakuum oder bei erhöhtem Druck (Überdruck) durchzuführen.

Die molaren Verhältnisse der Verbindung der Formel (**II**) zu Säuren der oben beschriebenen Gruppe kann in weiten Grenzen variiert werden. Üblicherweise arbeitet man mit einem molaren Verhältnis von 1:1 bis 1:3, vorzugsweise von 1:2 bis 1:3. Besonders bevorzugt wird ein molares Verhältnis von 1:2.

Die molaren Verhältnisse der Verbindung der Formel (**II**) zu Nitriten der oben beschriebenen Gruppe kann in weiten Grenzen variiert werden. Üblicherweise arbeitet man mit einem molaren Verhältnis von 1:1 bis 1:2, vorzugsweise von 1:1 bis 1:1.5. Besonders bevorzugt wird ein molares Verhältnis von 1:1.1.

Die Aniline der Formel (**II**) sind literaturbekannt und teilweise in technischen Mengen verfügbar (siehe beispielsweise Tetrahedron Lett. 2000, 41, 6319; Tetrahedron Lett. 1993, 34, 1057; J. Org. Chem. 1990, 55, 580).

Die Reaktionsdauer der Reaktion zur Bildung der Diazoniumsalz Verbindungen der Formel (**III**) ist kurz und liegt im Bereich von 0,5 bis 2 Stunden. Eine längere Reaktionsdauer ist möglich, jedoch wirtschaftlich nicht sinnvoll. Die Reaktionsdauer der Weiterreaktion der Verbindungen der Formel (**III**) zu Verbindungen der Formel (**I**) ist kurz und liegt im Bereich 0,5 bis 2 Stunden. Eine längere Reaktionsdauer ist möglich, jedoch wirtschaftlich nicht sinnvoll.

### Beispiele

Die vorliegende Erfindung wird anhand der nachfolgenden Beispiele näher erläutert, ohne die Erfindung dabei auf diese einzuschränken.

### 4-Chlor-2,3-dihydro-1-benzofuran (Ia)

Eine Lösung von 2-(2-Amino-6-chlorphenyl)ethanol (**IIa**) (17.2 g, 100.2 mmol) in 1,2-Dichlorethan (55 ml) wurde bei 0 - 5 °C mit Trifluoressigsäure (15.4 ml, 200.4 mmol) versetzt. *n*-Butylnitrit (95%, 13.6 ml, 110.2 mmol) wurde über einen Zeitraum von 1h bei einer Temperatur von 0 - 5 °C zudosiert und nach beendeter Zugabe 1h bei dieser Temperatur gerührt. Das entstandene Diazonium Salz wurde nicht isoliert und direkt weiter umgesetzt. Analytische Daten des Diazonium Trifluoracetat Salz (**IIIa**, X = F₃CCOO⁻) sind wie folgt: ¹H-NMR (DMSO-d6, 400 MHz) δ (ppm) = 8.71 (dd, *J* = 8.3, 1.2 Hz, 1H), 8.41 (dd, *J* = 8.3, 1.1 Hz, 1H), 7.90 (t, *J* = 8.3 Hz, 1H), 3.75 (t, *J* = 5.6 Hz, 2H), 3.33 (t, *J* = 5.6 Hz, 2H); ¹H-NMR (CDCl₃, 400 MHz) δ (ppm) = 8.70 (dd, *J* = 8.3, 1.0 Hz, 1H), 8.08 (dd, *J* = 8.3, 1.1 Hz, 1H), 7.63 (t, *J* = 8.3 Hz, 1H), 3.93 (t, *J* = 5.3 Hz, 2H), 3.37 (t, *J* = 5.3 Hz, 2H). Analytische Daten des Diazonium Trichloracetat Salz (**IIIa**, X = Cl₃CCOO⁻) sind wie folgt: ¹H-NMR (DMSO-d6, 400 MHz) δ (ppm) = 7.83 (dd, *J* = 8.0, 1.1 Hz, 1H), 7.62 (dd, *J* = 8.0, 1.1 Hz, 1H), 7.48 (t, *J* = 8.0 Hz, 1H), 3.57 (t, *J* = 7.1 Hz, 2H), 3.39 (t, *J* = 7.1 Hz, 2H). Analytische Daten des Diazonium Chlorid Salz (**IIIa**, X = Cl⁻) sind wie folgt: ¹H-NMR (DMSO-d6, 400 MHz) δ (ppm) = 8.81 (dd, *J* = 8.4, 1.1 Hz, 1H), 8.41 (dd, *J* = 8.2, 1.0 Hz, 1H), 7.91 (t, *J* = 8.3 Hz, 1H), 3.73 (t, *J* = 5.6 Hz, 2H), 3.33 (t, *J* = 5.6 Hz, 2H). Analytische Daten des Diazonium Hydrogensulfat Salz (**IIIa**, X = HSO₄⁻) sind wie folgt: ¹H-NMR (DMSO-d6, 400 MHz) δ (ppm) = 8.73 (dd, *J* = 8.3, 1.1 Hz, 1H), 8.40 (dd, *J* = 8.3, 1.1 Hz, 1H), 7.90 (t, *J* = 8.3 Hz, 1H), 3.74 (t, *J* = 5.6 Hz, 2H), 3.33 (t, *J* = 5.6 Hz, 2H).

Die obige Lösung wurde über einen Zeitraum von 1h zu 1,2-Dichlorethan (30 ml) bei 70 - 80 °C zudosiert und nach beendeter Zugabe weitere 30min bei dieser Temperatur gerührt. Die Reaktionslösung wurde auf 20 °C abgekühlt und mit 10% HCl Lösung gewaschen (20 ml). Die organische Phase wurde über Magnesiumsulfat getrocknet, filtriert und im Vakuum eingeengt. Der Rückstand wurde anschließend im Vakuum fraktioniert destilliert (Sdp. 89 - 91 °C / 10 mbar, 11.9 g, 77% der Theorie). ¹H-NMR (CDCl₃, 400 MHz) δ (ppm) = 7.04 (tt, *J* = 8.0, 0.7 Hz, 1H), 6.82 (dd, *J* = 8.0, 0.7 Hz, 1H), 6.67 (d, *J* = 8.1 Hz, 1H), 4.60 (t, *J* = 8.7 Hz, 2H), 3.25 (t, *J* = 8.7 Hz, 2H).

### 4-Brom-2,3-dihydro-1-benzofuran (Ib)

Sdp. 106 - 108 °C / 10 mbar; ¹H-NMR (DMSO-d6, 400 MHz) δ (ppm) = 7.05 (t, *J* = 7.7 Hz, 1H), 7.01 (dd, *J* = 8.1, 1.1 Hz, 1H), 6.76 (dd, *J* = 7.6, 1.1 Hz, 1H), 4.58 (t, *J* = 8.7 Hz, 2H), 3.17 (t, *J* = 8.7 Hz, 2H).

### 4-Methyl-2,3-dihydro-1-benzofuran (Ic)

Sdp. 89 - 91 °C / 10 mbar; ¹H-NMR (DMSO-d6, 400 MHz) δ (ppm) = 6.96 (t, *J* = 7.7 Hz, 1H), 6.63 (d, *J* = 7.6 Hz, 1H), 6.55 (d, *J* = 8.0 Hz, 1H), 4.50 (t, *J* = 8.7 Hz, 2H), 3.08 (t, *J* = 8.7 Hz, 2H), 2.19 (s, 3H).

### Vergleichsbeispiel:

### Herstellung von 4-Chlor-2,3-dihydro-1-benzofuran (Ia) unter Erhalt von 3-Chlor-2-(2-hydroxy-ethyl)phenol als Nebenkomponente

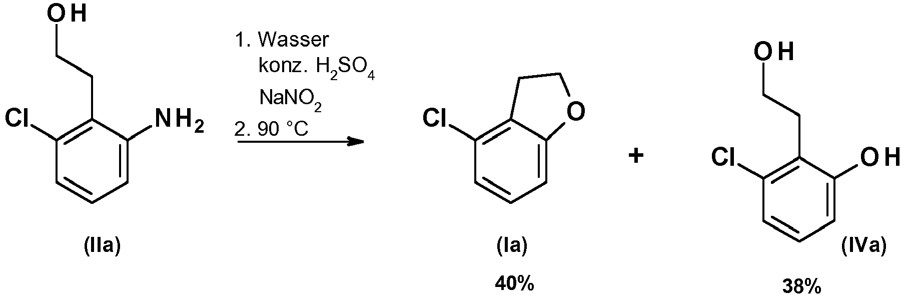

2-(2-Amino-6-chlorphenyl)ethanol (**IIa**) (10.0 g, 58.3 mmol) wurde in einer Mischung aus Wasser (250 ml) und konzentrierter Schwefelsäure (25.0 ml) aufgenommen und auf 0 °C abgekühlt. Eine Lösung von Natriumnitrit (7.24 g, 104.9 mmol) in Wasser (50.0 ml) wurde über einen Zeitraum von 1h bei einer Temperatur von 0 - 5 °C zudosiert und nach beendeter Zugabe 1h bei 0 °C nachgerührt. HPLC zeigte vollständigen Umsatz zum Diazonium Salz. Das entstandene Diazonium Salz wurde nicht isoliert und direkt weiter umgesetzt.

Die obige Lösung wurde über einen Zeitraum von 1h zu Wasser (50 ml) bei 80 - 90 °C zudosiert und nach beendeter Zugabe weitere 30 min bei dieser Temperatur gerührt. HPLC zeigt vollständigen Umsatz des Diazonium Salz zu 4-Chlor-2,3-dihydro-1-benzofuran (**Ia**, 41Fl% HPLC)) und 3-Chlor-2-(2-hydroxyethyl)phenol (**IVa**, 56Fl% HPLC). Das Reaktionsgemisch wurde auf 20 °C abgekühlt und dreimal mit Ethylacetat extrahiert (je 100 ml). Die vereinigten organischen Phasen wurden dreimal mit 5% NaOH gewaschen (je 100 ml). Die verbliebene organische Phase wurde mit gesättigter Natriumchlorid Lösung gewaschen, über MgSO₄ getrocknet und am Rotationsverdampfer eingeengt. Man erhielt 4-Chlor-2,3-dihydro-1-benzofuran (**Ia**) als orange-gelbliches Öl (4.0 g, Reinheit: 91Fl% HPLC, Ausbeute: 40% der Theorie, Analytik Daten: siehe erfindungsgemäßes Beispiel). Die vereinigten, wässrigen NaOH Phasen wurden durch Zugabe von 20% HCl auf einen pH-Wert von 1-2 gebracht und anschließend dreimal mit EtOAc (je 100 ml) extrahiert. Die vereinigten organischen Phasen wurden mit gesättigter Natriumchlorid Lösung gewaschen, über MgSO₄ getrocknet und am Rotationsverdampfer eingeengt. Man erhielt 3-Chlor-2-(2-hydroxyethyl)phenol (**IVa**) als orange-gelbliches Öl (4.0 g, Reinheit: 96Fl% HPLC, Ausbeute: 38% der Theorie) mit folgenden analytischen Daten: ¹H-NMR (CDCl₃, 400 MHz) δ (ppm) = 7.07 (t, *J* = 8.1 Hz, 1H), 6.97 (d, *J* = 8.1 Hz, 1H), 6.85 (d, *J* = 8.1 Hz, 1H), 4.01 (t, *J* = 8.5 Hz, 2H), 3.12 (t, *J* = 8.5 Hz, 2H).

## Patentansprüche

1. Verfahren zur Herstellung von substituierten 2,3-Dihydro-1-benzofuran-Derivaten der Formel (**I**): 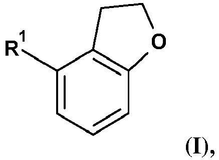 in der
R¹ für Cl (**Ia**), Br (**Ib**) oder Methyl (**Ic**) steht,
**dadurch gekennzeichnet dass** ein Anilin der Formel (**II**) 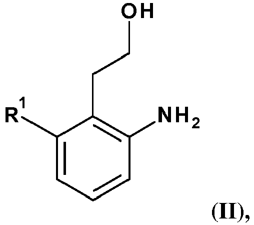 in der
R¹ für Cl, Br oder Methyl steht, in Gegenwart von organischem Nitrit und organischen oder anorganischen Säuren in organischen Lösungsmitteln zum Diazoniumsalz der Formel (**III**) umgesetzt wird, 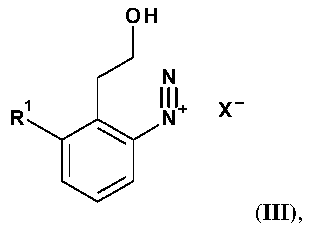 in der
R¹ für Cl, Br oder Methyl steht,
X⁻ für das Gegenion der organischen oder anorganischen Säure steht,
das durch Erhitzen zu Verbindungen der Formel (**I**) weiterreagiert.

2. Verfahren nach Anspruch 1, **dadurch gekennzeichnet, dass**
R¹ für Cl steht.

3. Verfahren nach einem der Ansprüche 1 oder 2, **dadurch gekennzeichnet, dass** X⁻ für Cl⁻, HSO₄⁻, Cl₃COO⁻ oder F₃CCOO⁻ steht.

4. Verfahren nach einem der Ansprüche 1 bis 3, **dadurch gekennzeichnet, dass** als Lösungsmittel Nitrile, Kohlenwasserstoffe und/oder halogenierte Kohlenwasserstoffe verwendet werden.

5. Verfahren nach einem der Ansprüche 1 bis 3, **dadurch gekennzeichnet, dass** als Lösungsmittel Dichlormethan, 1,2-Dichlorethan, 1-Chlorbutan, Toluol, Xylol, Chlorbenzol, 1,2-Dichlorbenzol und/oder Acetonitril verwendet werden.

6. Verfahren nach einem der Ansprüche 1 bis 5, **dadurch gekennzeichnet, dass** als organisches Nitrit ein C₁-C₆-Alkylnitrit verwendet wird.

7. Verfahren nach einem der Ansprüche 1 bis 6, **dadurch gekennzeichnet, dass** eine der folgenden Kombinationen aus Lösungsmittel, organischem Nitrit und Säure für die Reaktion verwendet wird:
a) ein oder mehrere Nitrile als Lösungsmittel, ein oder mehrere C₁-C₆-Alkylnitrite als organisches Nitrit und Schwefelsäure oder Salzsäure als Säure;
b) Kohlenwasserstoffe und/oder halogenierte Kohlenwasserstoffe als Lösungsmittel, ein oder mehrere C₁-C₆-Alkylnitrite als organisches Nitrit und Trifluoressigsäure oder Trichloressigsäure als Säuren.

8. Verfahren nach einem der Ansprüche 1 bis 3, **dadurch gekennzeichnet, dass** eine der folgenden Kombinationen aus Lösungsmittel, organischem Nitrit und Säure für die Reaktion verwendet wird:
a) Acetonitril, Propionitril und/oder Butyronitril in Kombination mit Schwefelsäure oder Salzsäure in Kombination mit n-Butylnitrit, tert-Butylnitrit und/oder Isopentylnitrit;
b) 1,2-Dichlorethan, Dichlormethan, 1-Chlorbutan, Toluol, Xylol, Chlorbenzol und/oder 1,2-Dichlorbenzol in Kombination mit Trifluoressigsäure oder Trichloressigsäure in Kombination mit n-Butylnitrit, tert-Butylnitrit und/oder Isopentylnitrit.

## Claims

1. Method for preparing substituted 2,3-dihydro-1-benzofuran derivatives of the formula **(I)**: 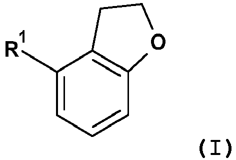 in which
R¹ is Cl (**Ia**), Br (**Ib**) or methyl (**Ic**), **characterized in that** an aniline of the formula **(II)** 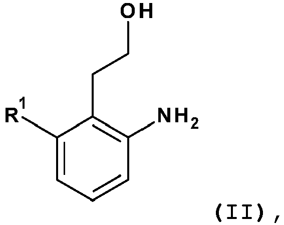 in which
R¹ is Cl, Br or methyl, is reacted in the presence of organic nitrite and organic or inorganic acids in organic solvents to give the diazonium salt of the formula **(III)**, 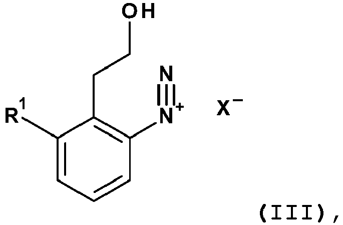 in which
R¹ is Cl, Br or methyl,
X⁻ is the counterion of the organic or inorganic acid,
which further reacts by heating to give compounds of the formula (**I**).

2. Method according to Claim 1, **characterized in that** R¹ is Cl.

3. Method according to either of Claims 1 and 2, **characterized in that** X⁻ is Cl⁻, HSO₄⁻, Cl₃COO⁻ or F₃CCOO⁻.

4. Method according to any of Claims 1 to 3, **characterized in that** nitriles, hydrocarbons and/or halogenated hydrocarbons are used as solvent.

5. Method according to any of Claims 1 to 3, **characterized in that** the solvents used are dichloromethane, 1,2-dichloroethane, 1-chlorobutane, toluene, xylene, chlorobenzene, 1,2-dichlorobenzene and/or acetonitrile.

6. Method according to any of Claims 1 to 5, **characterized in that** the organic nitrite used is a C₁-C₆-alkyl nitrite.

7. Method according to any of Claims 1 to 6, **characterized in that** one of the following combinations of solvent, organic nitrite and acid is used for the reaction:
a) one or more nitriles as solvent, one or more C₁-C₆-alkyl nitrites as organic nitrite and sulfuric acid or hydrochloric acid as acid;
b) hydrocarbons and/or halogenated hydrocarbons as solvent, one or more C₁-C₆-alkyl nitrites as organic nitrite and trifluoroacetic acid or trichloroacetic acid as acids.

8. Method according to any of Claims 1 to 3, **characterized in that** one of the following combinations of solvent, organic nitrite and acid is used for the reaction:
a) acetonitrile, propionitrile and/or butyronitrile in combination with sulfuric acid or hydrochloric acid in combination with n-butyl nitrite, tert-butyl nitrite and/or isopentyl nitrite;
b) 1,2-dichloroethane, dichloromethane, 1-chlorobutane, toluene, xylene, chlorobenzene and/or 1,2-dichlorobenzene in combination with trifluoroacetic acid or trichloroacetic acid in combination with n-butyl nitrite, tert-butyl nitrite and/or isopentyl nitrite.

## Revendications

1. Procédé pour la préparation de dérivés de 2,3-dihydro-1-benzofuranne substitués de formule (I) : 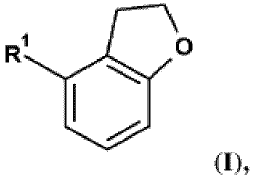 dans laquelle
R¹ représente Cl (Ia), Br (Ib) ou méthyle (Ic), **caractérisé en ce qu'**une aniline de formule (II) 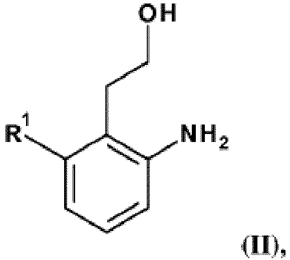 dans laquelle
R¹ représente Cl, Br ou méthyle, est transformée en présence d'un nitrite organique et d'acides organiques ou inorganiques, dans des solvants organiques, en sel de diazonium de formule (III) 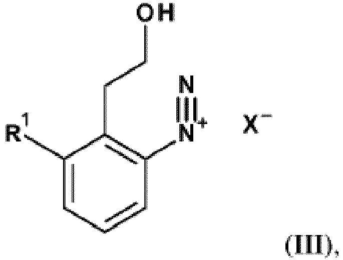 dans laquelle
R¹ représente Cl, Br ou méthyle,
X⁻ représente le contre-ion de l'acide organique ou inorganique,
qui, par chauffage continue de réagir pour donner des composés de formule (I).

2. Procédé selon la revendication 1, **caractérisé en ce que** R¹ représente Cl.

3. Procédé selon l'une quelconque des revendications 1 et 2, **caractérisé en ce que** X⁻ représente Cl⁻, HSO₄⁻, Cl₃COO⁻ ou F₃CCOO⁻.

4. Procédé selon l'une quelconque des revendications 1 à 3, **caractérisé en ce que** des nitriles, des hydrocarbures et/ou des hydrocarbures halogénés sont utilisés en tant que solvant.

5. Procédé selon l'une quelconque des revendications 1 à 3, **caractérisé en ce que** du dichlorométhane, du 1,2-dichloroéthane, du 1-chlorobutane, du toluène, du xylène, du chlorobenzène, du 1,2-dichlorobenzène et/ou de l'acétonitrile sont utilisés en tant que solvant.

6. Procédé selon l'une quelconque des revendications 1 à 5, **caractérisé en ce qu'**un C₁-C₆-alkylnitrite est utilisé en tant que nitrite organique.

7. Procédé selon l'une quelconque des revendications 1 à 6, **caractérisé en ce qu'**une des combinaisons suivantes de solvant, de nitrite organique et d'acide est utilisée pour la réaction :
a) un ou plusieurs nitriles en tant que solvant, un ou plusieurs C₁-C₆-alkylnitrites en tant que nitrite organique et de l'acide sulfurique ou de l'acide chlorhydrique en tant qu'acide ;
b) des hydrocarbures et/ou des hydrocarbures halogénés en tant que solvant, un ou plusieurs C₁-C₆-alkylnitrites en tant que nitrite organique et de l'acide trifluoroacétique ou de l'acide trichloroacétique en tant qu'acides.

8. Procédé selon l'une quelconque des revendications 1 à 3, **caractérisé en ce qu'**une des combinaisons suivantes de solvant, de nitrite organique et d'acide est utilisée pour la réaction :
a) de l'acétonitrile, du propionitrile et/ou du butyronitrile en combinaison avec de l'acide sulfurique ou de l'acide chlorhydrique en combinaison avec du n-butylnitrite, du tert-butylnitrite et/ou de l'isopentylnitrite ;
b) du 1,2-dichloroéthane, du dichlorométhane, du 1-chlorobutane, du toluène, du xylène, du chlorobenzène et/ou du 1,2-dichlorobenzène en combinaison avec de l'acide trifluoroacétique ou de l'acide trichloroacétique en combinaison avec du n-butylnitrite, du tert-butylnitrite et/ou de l'isopentylnitrite.
